(19) Europäisches Patentamt
European Patent Office
Office européen des brevets

(11) Publication number : **0 556 957 A1**

(12) # EUROPEAN PATENT APPLICATION

(21) Application number : **93300449.1**

(51) Int. Cl.$^5$ : **A61K 7/48**

(22) Date of filing : **21.01.93**

(30) Priority : **23.01.92 GB 9201476**
**22.12.92 GB 9226690**

(43) Date of publication of application :
**25.08.93 Bulletin 93/34**

(84) Designated Contracting States :
**AT BE CH DE DK ES FR GB GR IE IT LI NL PT SE**

(71) Applicant : **UNILEVER PLC**
**Unilever House Blackfriars**
**London EC4P 4BQ (GB)**
(84) **GB IE**

(71) Applicant : **UNILEVER N.V.**
**Weena 455**
**NL-3013 AL Rotterdam (NL)**
(84) **BE CH DE DK ES FR GR IT LI NL PT SE AT**

(72) Inventor : **Critchley, Peter**
**Unilever Research Colworth Lab., Colworth House**
**Sharnbrook, Bedford MK44 1LQ (GB)**

(74) Representative : **Tonge, Robert James**
**UNILEVER PLC Patent Division Colworth House Sharnbrook**
**Bedford MK44 1LQ (GB)**

(54) **Cosmetic composition for treating dry skin.**

(57) A cosmetic composition effective in the reduction of water loss through the stratum corneum comprising three components A, B and C, wherein A is a molecule having at least two hydrocarbon chains and a polar head group for which :

$$\frac{V}{a_o l_c} > 0.5 \leqq 1.0 ;$$

Component B is a molecule having one long hydrocarbon chain and a polar head group ; and component C is capable of stabilising any lipid bilayers assisting the formulation of lipid bilayers and formed in the composition ; providing that the molar ratio of A :B :C is 1 :1.5 to 6.0 :1.1 to 8.0.

EP 0 556 957 A1

## FIELD OF THE INVENTION

This invention relates to cosmetic compositions and particularly compositions concerned with the prevention or amelioration of skin wrinkling, chapping or ageing.

## BACKGROUND TO THE INVENTION AND PRIOR ART

It is generally understood that ceramides present within the intercellular lipid lamellae of the stratum corneum play an important role in the production and maintenance of the water permeability barrier of the skin. Ceramides, or substances closely related to them, have been disclosed as components of skin care compositions. In particular, Kao Corporation in GB 2 178 312 and GB 2 213 723 discloses the use of natural ceramides extracted from skin in products for topical application. Also, Kao Corporation in EP 227 994 and EP 282 816 discloses the use of synthetic ceramides, which are similar to their natural counterparts found in skin.

It is also known that in addition to ceramides the lipid lamellae comprises sterols and fatty acids N Y Schurer, P M Elias (1991) Adv Lip Res 24 27-56 Acad Press.

It is believed that one of the causes of dry skin and ageing skin is a reduction in the amount of lipid contained within these intercellular lipid lamellae. It is therefore desirable to be able to successfully replace these depleted lipids by the topical route.

One suitable method for testing the ability of cosmetic compositions to effect the desired repair of the water barrier layer (ie the intercellular lipid lamellae) is to study the ability of the cosmetic composition to reduce water loss through the stratum corneum. Although previously proposed cosmetic compositions have shown a nominal level of water loss reduction, the composition according to the present invention shows a significantly improved ability for such a water loss reduction and hence provides a much more effective control of water loss and/or repair of damage to the water barrier layer in the stratum corneum.

It has been shown in Chapter 16 entitled "Aggregation of Amphiphilic Molecules into Micelles, Bilayers, Vesicles and Biological Membranes" in Intermolecular and Surface Forces, (1985) Jacob N Isrealachvili, ed Acad Press that suitable lipids for forming a bilayer are those having a polar head group and at least two hydrocarbon chains, such that there exists a clearly defined relationship between the volume occupied by the hydrocarbon chains and the optimum area occupied by the polar head group. This relationship is that:

$$\frac{V}{a_o l_c}$$

should be greater than 0.5 but less than or equal to 1.0

where

- $V$ is the volume of the hydrocarbon chains
- $l_c$ is the critical length of the hydrocarbon chains
- $a_o$ is the optimum area of the polar head group

## DEFINITION OF THE INVENTION

The invention provides a cosmetic composition comprising three components A, B and C, wherein A is a molecule having at least two hydrocarbon chains and a polar head group for which $\frac{V}{a_o l_c} > 0.5 \leqq 1.0$; component B is a molecule having one long hydrocarbon chain and a polar head group; and component C is capable of assisting the formation of lipid bilayers and stabilising any lipid bilayers formed in the composition; providing that the molar ratio of A:B:C is 1:1.5 to 6.0:1.1 to 8.0.

## DISCLOSURE OF THE INVENTION

The invention provides a composition suitable for topical application to the skin. The site of action of the composition is in the stratum corneum of the skin, and its mode of action is to effectively control water loss and/or to repair damage to the water barrier layer in the stratum corneum.

### Component A

Component A is a molecule having at least two hydrocarbon chains and a polar head group, providing that the volume occupied by the hydrocarbon chains and the area occupied by the polar head group fulfils the re-

lationship $\frac{V}{a_o l_c} > 0.5 \leqq 1.0$

where V, $l_c$, and $a_o$ are as defined above.

Preferably the hydrocarbon chains each have at least 14 carbon atoms. Furthermore it is preferred that hydrocarbon chains having less than 16 carbon atoms are fully saturated, however, hydrocarbon chains having more than 16 carbon atoms may contain 1 to 3 unsaturations if desired.

Suitable polar head groups may be selected from residues such as phosphates, phosphonates, sulphates, sulphonates, sulphones, hydroxyl, ethylene oxide, carboxyl and mixtures thereof. Preferably the polar head group is selected from hydroxyl groups, ethylene oxide units, carboxyl groups and mixtures thereof.

Suitable compounds that may be selected as component A are ceramides; pseudoceramides; phospholipids; glycolipids having a structure of two or more acyl or alkyl long chains suitably containing from 14 to 50 carbon atoms each, attached to a polar group; specific esters of polyethylene glycol; polyglycerol -n-x oleate (CAS 9007-48-1); sorbitan dioleate (CAS 29116-98-1); sorbitan sesquioleate (CAS 8007-43-0); long chain alkyl ether versions of phospholipids and glycolipids; and mixtures thereof.

<u>Ceramides</u>

Ceramides are preferably selected from ceramides having the general structure (1)

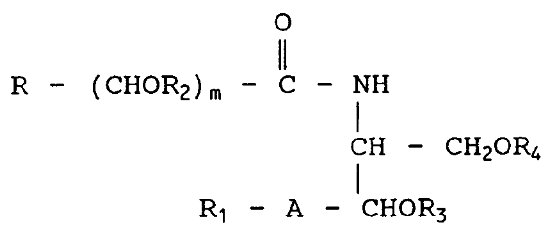

(1)

where A represents $-CH_2-$; $-CHOR_5-$; $-CH=CH-$ or $-CHOY-$

R represents a linear or branched, saturated or unsaturated aliphatic hydrocarbon group having from 1 to 49 carbon atoms, preferably from 12 to 49 carbon atoms, or a subgroup (2).

$$Y-O-(C_aH_b)- \quad (2)$$

$R_1$ represents a linear or branched, saturated or unsaturated aliphatic hydrocarbon group having from 8 to 28 carbon atoms, preferably from 13 to 28 carbon atoms;

$R_2$, $R_3$ and $R_5$ individually represent H, a phosphate residue or a sulphate residue;

$R_4$ represents H, a phosphate residue, a sulphate residue or a sugar residue;

a is an integer of from 7 to 49, preferably from 12 to 49

b is an integer of from 10 to 98, preferably from 24 to 98

m is 0 or 1

Y represents H or a residue of a $C_{14-22}$ fatty acid having the general structure (3)

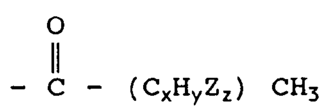

(3)

where

Z is - OH or an epoxy oxygen

x is an integer of from 12 to 20

y is an integer of from 20 to 40

and

z is 0 or an integer of from 1 to 4

Ceramides having the general structure (1) are naturally occurring and can be isolated from a suitable plant source or from animal tissue such as pig skin or neural tissue. Ceramides can also be synthesised.

Particular preferred examples of ceramides are ceramide I and ceramide II.

Pseudoceramides

Pseudoceramides are preferably selected from pseudoceramides (ie synthetic ceramide like structures) having the general structure (4):

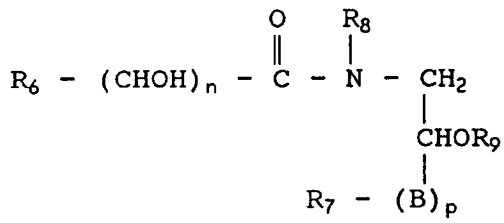

(4)

where B represents - $OCH_2$ - or CHOH.

$R_6$ represents a linear or branched, saturated or unsaturated aliphatic hydrocarbon group having from 1 to 49 carbon atoms preferably from 12 to 49 carbon atoms or the subgroup (2).

$R_7$ represents a linear or branched, saturated or unsaturated aliphatic hydrocarbon group having from 8 to 28 carbon atoms preferably from 13 to 28 carbon atoms.

$R_8$ represents H, or a subgroup - $(CH_2)_cCOOH$, where c is an integer of from 1 to 6, or a subgroup having the structure (5).

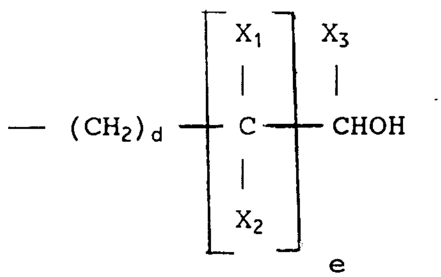

(5)

where $X_1$, $X_2$ and $X_3$ each individually represent H, a $C_{1-5}$ alkyl or a $C_{1-5}$ hydroxyalkyl;

d is 0 or an integer of from 1 to 4

e is 0 or 1

n is 0 or 1

and

p is 0 or 1;

$R_9$ represents H, a phosphate residue, a sulphate residue or a sugar residue.

Phospholipids

Phospholipids may be advantageously used because they are derivable from plant sources. An example of a suitable phospholipid material is a mixture of phospholipids derived from a particular fraction in a continuous soya bean phospholipid extraction and is sold under the trade name Ceramax by Quest International of Ashford, Kent, UK.

Glycolipids

Suitable glycolipids are those having a structure of two or more acyl or alkyl long chains suitably containing from 14 to 50 carbon atoms each, attached to a polar head group. The polar head group may be selected from residues such as phosphates, phosphonates, sulphates, sulphonates, sulphones, hydroxyl, ethylene oxide, carboxyl and mixtures thereof. Preferably the polar head group is hydroxylated.

Suitable examples are glycosyl glycerides or diacyl or dialkyl saccharides, eg sucrose diesters with two or more long chain ester groups. An example of the latter type of material is obtainable from Ryoto under the appellation Ryoto sugar esters such as Ryoto sugar ester S270, and S1570.

Esters of Polyethylene Glycol

Suitable esters are:

(i) succinic acid esters having the general structure (6)

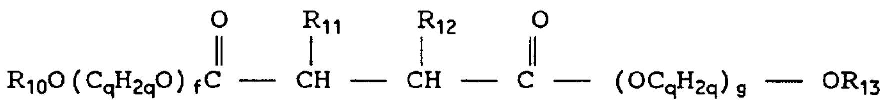

(6)

in which $R_{10}$ represents an alkyl, alkenyl, mono- or dihydroxyalkyl or hydroxyalkenyl group having from 6-22 carbon atoms;

$R_{11}$ and $R_{12}$ individually represent H or an alkyl or alkenyl group having from 12 to 22 carbon atoms; providing that when $R_{11}$ is H, $R_{12}$ is an alkyl or alkenyl group and when $R_{12}$ is H, $R_{11}$ is an alkyl or alkenyl group;

$R_{13}$ represents hydrogen, an alkyl, alkenyl, mono- or dihydroxyalkyl or hydroxyalkenyl group having from 6 to 22 carbon atoms or the group (7):

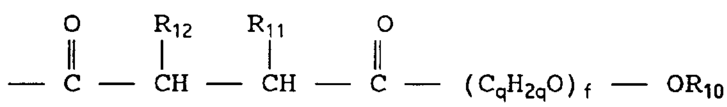

(7)

q is an integer of from 2 to 3

f and g are average degrees of alkoxylation, namely f is from 0 to 20 and g is from 1 to 20.

In structure (6), the group $R_{13}$ preferably represents H, while the group $R_{10}$ preferably represents an alkyl group having from 16 to 22 carbon atoms and most preferably from 20 to 22 carbon atoms.

Also with reference to structure (6), q is preferably 2 and (f+g) is preferably from 1 to 20.

Such succinic acid esters are synthesised using known methods of preparative chemistry, these methods are described in our co-pending patent application GB 9223578.7 filed 11 November 1992.

(ii) Polyethylene glycol r-dilaurate (CAS 9005-02-1) and polyethylene glycol r-distearate (CAS 9005-08-7).

(iii)Polyethylene glycol r-distearammonium chlorides. These may be derived from polyethylene glycol r-tallow amine (CAS 61791) by alkylating the nitrogen with an alkyl halide having more than 8 carbon atoms.

(iv) Polyethylene glycol derivatives of long chain alkyl derivatives of malic, tartaric, maleic, malonic and glyceric acid.

where r is the number of ethylene oxide groups within the molecule and is preferably from 1 to 8, most preferably from 2 to 4.

Preferably component A comprises a mixture of the above mentioned compounds. More preferably this mixture is such that a range of chain lengths are present. Most preferred are mixtures of ceramide and phospholipids, particularly the phospholipid mixture having the trade name Ceramax, wherein the ratio of ceramide to Ceramax is from 1:1000 to 1:1, preferably from 1:50 to 1:1 and most preferably from 1:10 to 1:5 by weight.

Component B

Component B is a molecule having one long hydrocarbon chain and a polar head group. A wide variety of simple co-surfactants are functional. Preferred co-surfactants are straight-chained mono carboxylic acids having 14 to 30 carbon atoms, straight-chained fatty alcohols having 14 to 30 carbon atoms, sugar esters, alkylated sugars and mixtures thereof.

Examples of suitable sugar esters and alkylated sugars are monopalmitoyl or 6-cetyl glucose and $\alpha$ or $\beta$ methyl 6-cetyl glucoside.

Preferably the co-surfactants are chosen from straight chained mono-carboxylic acids having 14 to 24 carbon atoms, fatty alcohols having 14 to 24 carbon atoms and mixtures thereof.

The mono-carboxylic acids may be used as 50-100% sodium or potassium soap.

A particularly preferred component B is linoleic acid, since linoleic acid assists in the absorption of ultraviolet light and furthermore is a vital component of the natural skin lipids constituting the moisture barrier in the stratum corneum.

Component B may comprise a mixture of compounds, for example a fatty acid mixture of palmitic, oleic and stearic acid at 3:2:1 by weight may be employed, however particularly useful fatty acids from the point of

view of availability are linoleic and stearic acid.

Component C

Component C is a compound capable of stabilising any lipid bilayers which may be formed in the composition.

Suitable compounds may be selected from 3β-sterols; squalene; squalane; saponins or sapogenins of the plant steroid or triterpenoid type; di and tri terpenes such as phytol, retinol and amyrin; and mixtures thereof.

Preferably component C is a 3β-sterol having a tail on the 17 position and having no polar groups, for example cholesterol, sitosterol, stigmasterol and ergosterol.

A particularly preferred component C is the 3β-sterol ergosterol since this is well known to convert in situ into vitamin $D_2$ (calciferol) on reception of ultraviolet light.

It is essential that the molar ratio in which the components A, B and C are present is from 1:1.5 to 6.0:1.1 to 8.0 respectively, in order to ensure adequate control of water flux.

Preferably components A, B and C are present from 1:1.5 to 4.0:1.1 to 4.0 respectively and most preferably from 1:1.8 to 3.6:1.2 to 3.0.

The composition according to the invention may include a cosmetically acceptable vehicle to act as dilutant, dispersant or carrier for the components. Suitable vehicles include water, squalene, squalane, volatile silicone, liquid or solid emollients, solvents, humectants, thickeners and powders.

Preferably the composition includes up to 5% by weight humectant, either as the vehicle or in addition to another vehicle, most preferably the humectant is glycerol.

Examples of particular mixtures include:

(i)
- A - Distearyl lecithin phospholipid
- B - Octadecanol
- C - Stigmasterol

(ii)
- A - Ceramide II (having two $C_{16}$ chains)
- B - a long chain fatty acid of $C_{16}$ to $C_{18}$
- C - cholesterol

Use of the Composition

The composition according to the invention is intended primarily as a product for topical application to human skin, especially as an agent for reducing the permeability of the skin to water, particularly when the skin is dry or damaged, in order to reduce moisture loss and generally to enhance the quality and flexibility of the skin. The composition can also be applied to hair and nails.

The composition may be used as a product for topical application to human skin to treat dry or ageing skin.

In use, a small quantity of the composition, for example from 1 to 5 ml, is applied to exposed areas of the skin, from a suitable container or applicator and, if necessary, it is then spread over and/or rubbed into the skin using the hand or fingers or a suitable device.

Examples

The invention is illustrated by the following examples.

Examples 1-15; Comparative Examples A-F

Compositions formulated according to the invention were tested to measure the reduction in water flux by the following method.

In Vitro Measurement of Water Vapour Transmission Rate

The reduction in water permeability of the skin following topical application of the composition according to the invention can be determined by in vitro measurement of the water vapour transmission rate (WVTR) using a water transmission cell adapted from that described by Blank I H, J Invest Dermatol, [1952], 18, 433-440.

Pretreatment of Porcine Stratum Corneum

Isolated porcine stratum corneum was floated on propan-2-ol contained in a glass petri dish. The dish was gently agitated for 1 hour at 40°C and the sample of extracted stratum corneum was then removed, floated in saline solution onto spectra mesh and air dried overnight.

Measurement of Initial WVTR Prior to Treatment

750 µl distilled water was placed in the centre well of the cell and a sample of pretreated stratum corneum (see above) was carefully laid onto a stainless steel grid over the well ensuring that the stratum corneum completely covered the O-ring, such that a watertight seal was achieved. Care was taken to avoid wrinkles, tears and holes in the stratum corneum sample. The top of the transmission cell was then screwed into position and allowed to equilibrate at room temperature before an initial measurement was made. The cell was weighed after 5 minutes, then placed in an incubator at 37°C, 5% RH. Two further weight measurements were taken at suitable intervals over a period of 24 hours at the end of which time a test or control solution was applied and three more measurements were taken during a further 21 hours. Five cells were used for each test or control treatment.

Study of the Effect of Topical Application of Test Material

For each test, a solution of test material in chloroform/methanol (2:1 v/v) was prepared at 15 mg/ml concentration. 15 µl of this solution was applied to the previously selected propan-2-ol extracted skin samples as described above. The chloroform/methanol quickly evaporated. The five cells containing the skin samples were weighed after 5 minutes prior to placing in the incubator at 37°C, 5% RH. As mentioned above, three weight measurements were then taken at intervals over a period of 21 hours.

A control measurement was made using other selected skin samples. This was carried out in the same way using an equal quantity of chloroform/methanol (2:1) containing no test material.

Calculation of the WVTR

The WVTR was calculated for each sample (pre and post topical application) as follows:

$$\text{WVTR (mg/cm}^2\text{/hr)} = \frac{\text{weight loss}}{\text{Area of exposed tissue x time}}$$

The mean WVTR for each group of cells was then calculated from these values. The standard deviation was calculated from the observed changes (relative increase or decrease) in WVTR measured before and after the topical application. It was then determined whether any observed reduction in WVTR was significant compared to the control measurement by use of the Duncan's Multiple Range Test.

Results are shown in table 1, structures mentioned in this table are as follows.

Structure 8

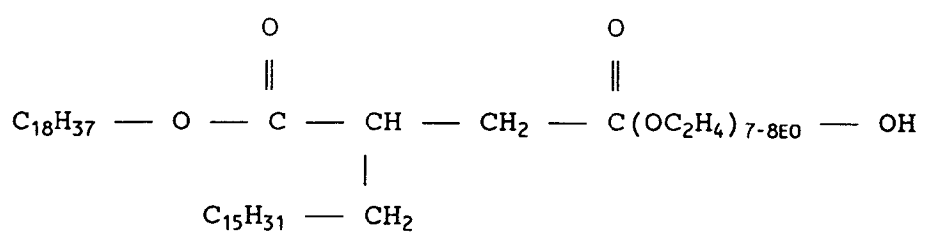

(8)

Structure 9

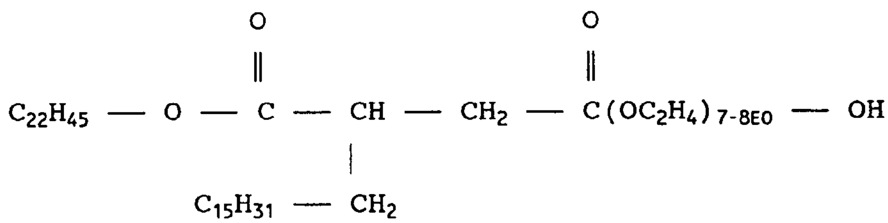

(9)

Structure 10

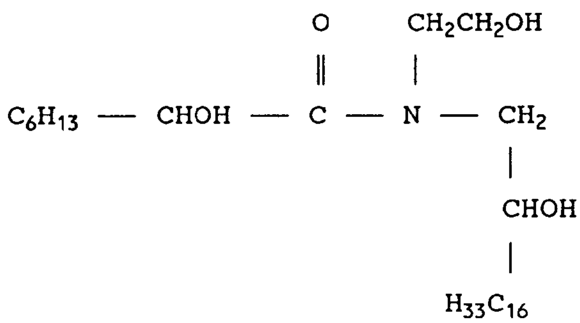

(10)

Structure 11

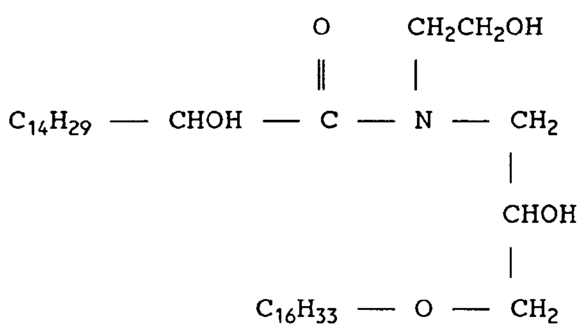

(11)

Structure 12

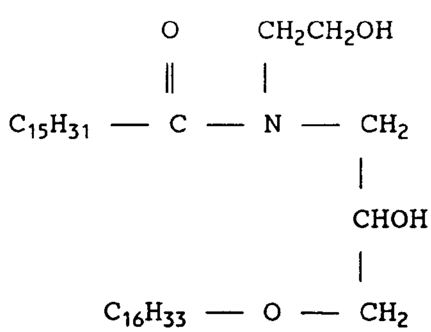

(12)

Table 1

| Example | Composition (molar ratio) | Significant reduction in WVTR compared with control |
|---|---|---|
| 1 | ceramide II : linoleic acid : cholesterol (1:2.1:2.7) | Yes |
| 2 | ceramide II : stearic acid : cholesterol (1:4.1:2.7) | Yes |
| 3 | ceramax : sodium stearate : cholesterol (1:2.9:3.8) | Yes |
| 4 | ceramax : stearic acid : cholesterol (1:5.9:3.8) | Yes |
| 5 | sucrose ester (S1570) : stearic acid : cholesterol (1:4.8:3.5) | Yes |
| 6 | sucrose ester (S270) : stearic acid : cholesterol (1:5.7:7.4) | Yes |
| 7 | structure 8 : stearic acid : cholesterol (1:5.8:4.2) | Yes |
| 8 | structure 9 : stearic acid : cholesterol (1:5.8:4.2) | Yes |
| 9 | structure 10 : stearic acid : cholesterol (1:4.3:3) | Yes |
| 10 | structure 11 : stearic acid : cholesterol (1:4.3:3) | Yes |

| Example | Composition (molar ratio) | Significant reduction in WVTR compared with control |
|---|---|---|
| 11 | ceramide : stearic acid : squalene (1:3.6:2.5) | Yes |
| 12 | ceramide : palmitic, oleic and stearic acid mixture (3:2:1 by weight) : squalene (1:2.1:1.25) | Yes |
| 13 | distearoyl lecithin phospholipid : octadecanol : stigmasterol (1:5.9:3.8) | Yes |
| 14 | ceramax/ceramide (5:1 by weight) : linoleic acid : ergosterol (1:2.16:3.04) | Yes |
| 15 | ceramide II : stearic acid : cholesterol (1:1.8:2.6) | Yes |
| A | ceramide II : isostearic acid : cholesterol (1:4.1:2.7) | No |
| B | structure 12 alone | No |
| C | ceramide II : fatty acid : squalane (1:1:1) | No |
| D | ceramax : cholesterol (1:1) | No |
| E | lecithin : cholesterol (2:1) | No |
| F | lecithin/ceramide (1:1) : cholesterol (2:1) | No |

## Claims

1. A cosmetic composition comprising three components A, B and C, wherein A is a molecule having at least two hydrocarbon chains and a polar head group for which:

$$\frac{V}{a_o l_c} > 0.5 \leqq 1.0;$$

component B is a molecule having one long hydrocarbon chain and a polar head group; and component C is capable of assisting the formation of lipid bilayers and stabilising any lipid bilayers formed in the composition; providing that the molar ratio of A:B:C is 1:1.5 to 6.0:1.1 to 8.0.

2. A cosmetic composition according to claim 1 wherein the molar ratio of A:B:C is 1:1.5 to 4.0:1.1 to 4.0.

3. A cosmetic composition according to claims 1 and 2 wherein the molar ratio of A:B:C is 1:1.8 to 3.6:1.2 to 3.0.

4. A cosmetic composition according to any preceding claim wherein the hydrocarbon chains of component A each have at least 14 carbon atoms.

5. A cosmetic composition according to any preceding claim wherein the polar head group of component A is a residue chosen from phosphates, phosphonates, sulphates, sulphonates, sulphones, hydroxyl, ethyl oxide, carboxyl and mixtures thereof.

6. A cosmetic composition according to any preceding claim wherein the polar head group of component A is chosen from hydroxyl groups, ethylene oxide units, carboxyl groups and mixtures thereof.

7. A cosmetic composition according to any preceding claim wherein component A is selected from ceramides; pseudoceramides; phospholipids; glycolipids having a structure of two or more acyl or alkyl long chains suitably containing from 14 to 50 carbon atoms attached to a polar head group; specific esters of polyethylene glycol; polyglycerol-n-x-oleate; sorbitan dioleate; sorbitan sesquioleate; long chain alkyl ether versions of phospholipids and glycolipids; and mixtures thereof.

8. A cosmetic composition according to any preceding claim wherein component A comprises a mixture of ceramide and Ceramax in the ratio of from 1:1000 to 1:1 by weight.

9. A cosmetic composition according to claim 8 wherein the ratio of ceramide to Ceramax is 1:50 to 1:1 by weight.

10. A cosmetic composition according to claims 8 and 9 wherein the ratio of ceramide to Ceramax is 1:10 to 1:5 by weight.

11. A cosmetic composition according to any preceding claim wherein component B is chosen from straight-chained mono carboxylic acids having 14 to 30 carbon atoms, straight chained fatty alcohols having 14 to 30 carbon atoms, sugar esters, alkylated sugars and mixtures thereof.

12. A cosmetic composition according to any preceding claim wherein component B is chosen from straight-chained mono-carboxylic acids having 14 to 24 carbon atoms, straight chained fatty alcohols having 14 to 24 carbon atoms and mixtures thereof.

13. A cosmetic composition according to any preceding claim wherein component B is chosen from linoleic acid, stearic acid and mixtures thereof.

14. A cosmetic composition according to any preceding claim wherein component C is selected from 3β-sterols; squalene; squalane; saponins or sapogenins of the plant sterol or triterpenoid type; di and tri terpenes; and mixtures thereof.

15. A cosmetic composition according to any preceding claim wherein component C is a 3β-sterol having a tail on the 17 position and no polar groups.

16. A cosmetic composition according to any preceding claim wherein the composition additionally comprises a cosmetically acceptable vehicle.

17. A cosmetic composition according to any preceding claim wherein the composition additionally comprises up to 5% by weight humectant.

18. A cosmetic composition according to claim 17 wherein the humectant is glycerol.

19. The use of a composition according to any preceding claim in the topical treatment of dry, ageing or detergent damaged human skin or hair.

European Patent Office

# EUROPEAN SEARCH REPORT

Application Number

EP 93 30 0449

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,P | EP-A-0 474 023 (KAO CORPORATION)<br>* claims 1-12; tables 1,3,5,7 *<br>--- | 1-19 | A61K7/48 |
| X | WO-A-9 001 323 (J. BERNSTEIN)<br>* claims 1-26 *<br>--- | 1-2,4-7, 11-19 | |
| A | PATENT ABSTRACTS OF JAPAN<br>vol. 12, no. 472 (C-551)(3319) 9 December 1988<br>& JP-A-63 192 703 ( KAO CORP ) 10 August 1988<br>* abstract *<br>--- | 1-19 | |
| A,D | EP-A-0 282 816 (KAO CORPORATION)<br>* page 5, line 37 - line 43; claims 1-8; examples 2-4,7,15-16 *<br>--- | 1-19 | |
| A,D | ADVANCES IN LIPID RESEARCH<br>vol. 24, 1991, NY, USA<br>pages 27 - 56<br>N. Y. SCHURER ET AL 'The Biochemistry and Function of Stratum Corneum Lipids'<br>----- | 1-19 | TECHNICAL FIELDS SEARCHED (Int. Cl.5)<br>A61K |

The present search report has been drawn up for all claims

| Place of search | Date of completion of the search | Examiner |
|---|---|---|
| BERLIN | 30 APRIL 1993 | SIATOU E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)